# EUROPÄISCHE PATENTANMELDUNG

(11) **EP 3 056 480 A1**
(43) Veröffentlichungstag der Anmeldung: **17.08.2016**
(21) Anmeldenummer: 15155233.8
(22) Anmeldetag: 16.02.2015
(51) Int. Cl.: C07C 2/84, C10G 9/36

(54) **Verfahren und Anlage zur Erzeugung von Kohlenwasserstoffen**

(71) Anmelder: Linde Aktiengesellschaft, 80331 München (DE)
(72) Erfinder: Zimmermann, Heinz, 81476 München (DE); Penner, Florian, 82538 Geretsried (DE)
(74) Vertreter: m patent group

(57) **Zusammenfassung**

Es wird ein Verfahren (100) zur Herstellung von Kohlenwasserstoffen vorgeschlagen, bei dem ein oder mehrere Dampfspalteinsatzströme (a), die überwiegend oder ausschließlich Kohlenwasserstoffe mit zwei und mehr Kohlenstoffatomen enthalten, unter Gewinnung eines oder mehrerer Dampfspaltabströme (b) einem oder mehreren Dampfspaltschritten (10) unterworfen werden, und bei dem ein oder mehrere Reaktionseinsatzströme (t, u), die überwiegend oder ausschließlich Methan enthalten, unter Gewinnung eines oder mehrerer Reaktionsabströme (v), die Ethan enthalten, einem oder mehreren Schritten (60) zur oxidativen Kopplung von Methan unterworfen werden, wobei unter Verwendung von Fluid des oder der Dampfspaltabströme (b) ein Trennabstrom (m), der überwiegend oder ausschließlich Ethan enthält, gebildet wird. Es ist bei dem vorgeschlagenen Verfahren vorgesehen, dass Fluid des oder der Reaktionsabströme (v) einem oder mehreren thermischen Spaltschritten (70) unterworfen wird, die dem oder den Schritten (60) zur oxidativen Kopplung von Methan nachgeschaltet sind, und in denen das Ethan, das in dem Fluid des oder der Reaktionsabströme (v) enthalten ist, unter dem Einfluss von Abwärme des oder der Schritte (60) zur oxidativen Kopplung von Methan zumindest teilweise zu Ethylen umgesetzt wird, und dass dem oder den thermischen Spaltschritten (70) Fluid (w) des Trennabstroms (m) zugeführt wird. Eine entsprechende Anlage ist ebenfalls Gegenstand der vorliegenden Erfindung.

## Beschreibung

Die Erfindung betrifft ein Verfahren und eine Anlage zur Erzeugung von Kohlenwasserstoffen gemäß den Oberbegriffen der unabhängigen Patentansprüche.

### Stand der Technik

Verfahren und Vorrichtungen zum Dampfspalten (engl. Steam Cracking) von Kohlenwasserstoffen sind bekannt und beispielsweise im Artikel "Ethylene" in Ullmann's Encyclopedia of Industrial Chemistry, online seit 15. April 2007, DOI 10.1002/14356007.a10_045.pub2, beschrieben. Beim Dampfspalten werden Kohlenwasserstoffe in Gegenwart von Dampf typischerweise bei Temperaturen von 800 bis 860 °C bei Verweilzeiten von ca. 0,1 bis 0,8 Sekunden gespalten.

Dampfspaltverfahren werden im kommerziellen Maßstab nahezu ausschließlich in beheizten Rohrreaktoren durchgeführt. Durch Dampfspaltverfahren werden dabei, wie allgemein bekannt, Gasgemische gewonnen, die neben den eigentlichen Zielverbindungen Nebenprodukte enthalten. Insbesondere werden in Dampfspaltverfahren typischerweise größere Mengen an Methan und Wasserstoff gebildet. Diese Komponenten der sogenannten Heizgasfraktion werden herkömmlicherweise einzeln oder gemeinsam als Heizgas zur Beheizung der genannten Rohrreaktoren verwendet.

Zugleich ist aber auch die stoffliche Nutzung von Methan durch aufwertende Reaktionen aus wirtschaftlicher Sicht von großem Interesse. So befinden sich derzeit Verfahren zur Herstellung höherer Kohlenwasserstoffe aus Methan durch oxidative Methankopplung (engl. Oxidative Coupling of Methane, OCM) in intensiver Entwicklung. Bei der oxidativen Methankopplung handelt es sich um die direkte Umsetzung von Methan in einem oxidativen, heterogen katalysierten Verfahren zu höheren Kohlenwasserstoffen. Entsprechende Verfahren erscheinen insbesondere zur Herstellung von Ethylen vielversprechend.

Bezüglich weiterer Details der oxidativen Methankopplung sei auf einschlägige Fachliteratur, beispielsweise Zavyalova, U. et al.: Statistical Analysis of Past Catalytic Data on Oxidative Methane Coupling for New Insights into the Composition of High-Performance Catalysts, ChemCatChem 3, 2011, 1935-1947, verwiesen.

Auch kombinierte Verfahren aus Dampfspaltverfahren und Verfahren zur Herstellung höherer Kohlenwasserstoffe aus Methan durch oxidative Methankopplung sind grundsätzlich bekannt. Es besteht der Bedarf nach einer verbesserten stofflichen Integration in derartigen Verfahren. Insbesondere sollen nach Möglichkeit in einem der beiden Verfahren anfallende Verbindungen in dem jeweils anderen Verfahren genutzt werden, falls dies aus wirtschaftlichen und/oder Energieeffizienzgründen von Vorteil ist.

### Offenbarung der Erfindung

Diese Aufgabe wird durch ein Verfahren und eine Anlage zur Erzeugung von Kohlenwasserstoffen mit den Merkmalen der unabhängigen Patentansprüche gelöst. Ausgestaltungen sind Gegenstand der abhängigen Patentansprüche sowie der nachfolgenden Beschreibung.

Vor der Erläuterung der Merkmale und Vorteile der vorliegenden Erfindung werden deren Grundlagen und die verwendeten Begriffe erläutert.

Dampfspaltverfahren werden, wie erwähnt, im kommerziellen Maßstab nahezu ausschließlich in Rohrreaktoren durchgeführt, in denen einzelne Reaktionsrohre (in Form von Rohrschlangen, sogenannten Coils) oder Gruppen von entsprechenden Reaktionsrohren auch bei unterschiedlichen Spaltbedingungen betrieben werden können. Unter gleichen oder vergleichbaren Spaltbedingungen betriebene Reaktionsrohre oder Gruppen von Reaktionsrohren, gegebenenfalls aber auch unter einheitlichen Spaltbedingungen betriebene Rohrreaktoren insgesamt, werden nachfolgend jeweils als "Spaltöfen" bezeichnet. Ein Spaltofen ist im hier verwendeten Sprachgebrauch also eine zum Dampfspalten verwendete bauliche Einheit, die einen Ofeneinsatz gleichen oder vergleichbaren Spaltbedingungen aussetzt. Eine Anlage zum Dampfspalten (auch als "Olefinanlage" bezeichnet) kann einen oder mehrere derartiger Spaltöfen aufweisen. Ein "Dampfspaltschritt" erfolgt im Sprachgebrauch der vorliegenden Anmeldung in einem oder mehreren Spaltöfen.

Mit dem Begriff "Ofeneinsatz" werden hier ein oder mehrere flüssige und/oder gasförmige Ströme bezeichnet, die einem oder mehreren Spaltöfen zugeführt werden. Auch durch ein entsprechendes Dampfspaltverfahren erhaltene Ströme, wie unten erläutert, können in einen oder mehrere Spaltöfen zurückgeführt und erneut als Ofeneinsatz verwendet werden. Als Ofeneinsatz eignet sich eine Vielzahl von Kohlenwasserstoffen und Kohlenwasserstoffgemischen von Ethan bis Gasöl bis zu einem Siedepunkt von typischerweise 600 °C.

Ein Ofeneinsatz kann aus einem sogenannten "Frischeinsatz" bestehen, also aus einem Einsatz, der anlagenextern bereitgestellt und beispielsweise aus einer oder mehreren Erdölfraktionen, Erdgas und/oder Erdgaskondensaten gewonnen wird. Ein Ofeneinsatz kann auch aus einem oder mehreren sogenannten "Recycleströmen" bestehen, also Strömen, die in der Anlage selbst erzeugt und in einen entsprechenden Spaltofen zurückgeführt werden. Ein Ofeneinsatz kann auch aus einem Gemisch eines oder mehrerer Frischeinsätze mit einem oder mehreren Recycleströmen bestehen.

Der Ofeneinsatz wird im jeweiligen Spaltofen zumindest teilweise umgesetzt und verlässt den Spaltofen als sogenanntes "Rohgas", das Nachbehandlungsschritten unterworfen werden kann. Derartige Nachbehandlungsschritte umfassen zunächst eine Aufbereitung des Rohgases, beispielsweise durch Quenchen, Kühlen und Trocknen, wodurch ein sogenanntes "Spaltgas" erhalten wird. Bisweilen wird auch bereits das Rohgas als Spaltgas bezeichnet.

Als allgemeine Bezeichnung für ein einem oder mehreren Spaltöfen zugeführtes Komponentengemisch, also einen oder mehrere Ofeneinsätze wie oben erläutert, wird nachfolgend auch der Begriff "Dampfspalteinsatzstrom", als allgemeine Bezeichnung für ein einem oder mehreren Spaltöfen entnommenes Gasgemisch nachfolgend auch der Begriff "Dampfspaltabstrom" verwendet.

Ein entsprechender Dampfspaltabstrom ist, wie erwähnt, ein Kohlenwasserstoffgemisch, das neben den gewünschten Zielverbindungen Nebenprodukte enthält. Ein Dampfspaltabstrom wird daher typischerweise zumindest teilweise in Fraktionen aufgetrennt. Dies kann mittels unterschiedlich ausgestalteter Trennsequenzen erfolgen, denen ein Dampfspaltabstrom beispielsweise nach einer sogenannten Rohgasverdichtung und weiteren Aufbereitungsschritten unterworfen wird. Entsprechende Trennsequenzen sind ebenfalls im erwähnten Artikel "Ethylene" in Ullmann's Encyclopedia of Industrial Chemistry erläutert.

Unter einem "Trennabstrom" wird jeder in einer entsprechenden Trennsequenz anfallende Strom verstanden. Ist dabei nachfolgend davon die Rede, dass "Fluid" eines Stroms (insbesondere Fluid eines Trennabstroms) für bestimmte Zwecke eingesetzt wird, kann dies die Verwendung des gesamten Stroms oder aber nur eines Teils davon oder nur bestimmter Komponenten umfassen. Enthält ein Trennabstrom oder ein anderer (Stoff-)Strom im Sprachgebrauch der vorliegenden Anmeldung "überwiegend" eine oder mehrere Komponenten, so ist bzw. sind diese Komponente(n) in einem Anteil von mindestens 50%, 60%, 70%, 80%, 90%, 95%, 99% oder 99,9% auf molarer, Volumen- oder Gewichts- bzw. Massebasis enthalten. Die Angabe, dass ein Strom "ausschließlich" eine oder mehrere Komponenten enthält, kann, muss aber nicht ausschließen, dass Spuren einer oder mehrerer anderer Komponenten enthalten sind. Als "Spuren" werden dabei beispielsweise Gehalte von weniger als 1%, 0,1%, 0,01% oder 0,001% auf molarer, Volumen- oder Gewichts- bzw. Massebasis betrachtet. Ist von einem "Methanstrom", einem "Ethanstrom", einem "Etylenstrom" oder dergleichen die Rede, sei hierunter ein Strom verstanden, der die jeweils bezeichnete Komponente überwiegend oder ausschließlich im erläuterten Sinn enthält.

### Vorteile der Erfindung

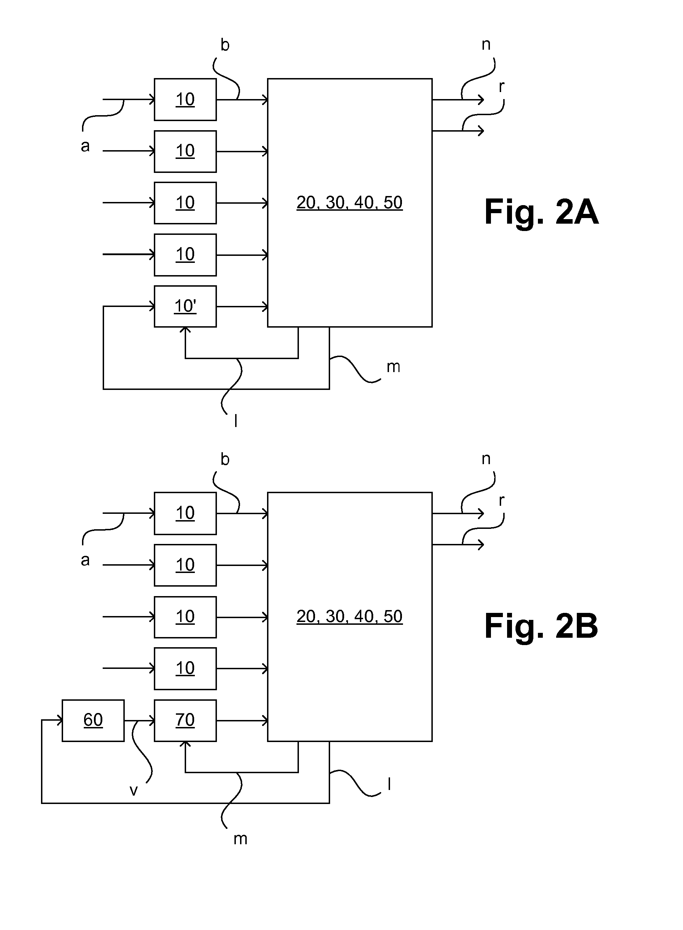

Die vorliegende Erfindung geht von einem Verfahren zur Erzeugung von Kohlenwasserstoffen aus, bei dem ein oder mehrere Dampfspalteinsatzströme, die überwiegend oder ausschließlich Kohlenwasserstoffe mit zwei und mehr Kohlenstoffatomen enthalten, unter Gewinnung eines oder mehrerer Dampfspaltabströme einem oder mehreren Dampfspaltschritten unterworfen werden. Eine derartige Anlage ist beispielsweise in der unten erläuterten Figur 2A dargestellt. Die in einem entsprechenden Verfahren durchgeführten Dampfspaltschritte können, wie erläutert, die Verwendung eines oder mehrerer, bei gleichen oder unterschiedlichen Bedingungen betriebener und/oder mit gleichen oder unterschiedlichen Einsätzen gespeister Spaltöfen umfassen. Beispielsweise können ein oder mehrere sogenannte Naphthaspaltöfen (also Spaltöfen für schwerere Frischeinsätze) und ein oder mehrere sogenannte Gasspaltöfen (also Spaltöfen für gasförmige Frischeinsätze) bereitgestellt sein.

Ferner umfasst das erfindungsgemäße Verfahren, einen oder mehrere Reaktionseinsatzströme, die überwiegend oder ausschließlich Methan enthalten, unter Gewinnung eines oder mehrerer Reaktionsabströme, die Ethan enthalten, einem oder mehreren Schritten zur oxidativen Kopplung von Methan zu unterwerfen. Verfahren zur oxidativen Kopplung von Methan wurden eingangs erläutert und sind dem Fachmann grundsätzlich bekannt.

Im Rahmen der vorliegenden Erfindung wird unter Verwendung von Fluid des oder der Dampfspaltabströme ein Trennabstrom gebildet, welcher überwiegend oder ausschließlich Ethan enthält. Ein derartiger Trennabstrom kann in bekannten Verfahren bzw. Trennsequenzen erzeugt werden, beispielsweise unter Verwendung von Demethanizern und Deethanizern sowie den bekannten C2-Splittern, wie sie in dem eingangs erwähnten Artikel "Ethylene" in Ullmann's Encyclopedia of Industrial Chemistry erläutert sind.

Erfindungsgemäß sind ein oder mehrere sogenannte Post-Bed-Cracking-Schritte vorgesehen, also ein oder mehrere thermische Spaltschritte, denen Fluid des oder der Reaktionsabströme der oxidativen Kopplung von Methan unterworfen wird, und in dem oder denen die Abwärme der oxidativen Kopplung von Methan zur Spaltung genutzt wird. Wie dem Fachmann grundsätzlich bekannt, fallen bei der oxidativen Kopplung von Methan große Mengen an Abwärme an, die sich aus der Exothermie der Reaktion ergeben. In dem oder den nachgeschalteten thermischen Spaltschritten wird dabei das Ethan, das in dem Fluid des oder der Reaktionsabströme enthalten ist und durch die oxidative Kopplung von Methan gebildet wurde, unter dem Einfluss der Abwärme des oder der Schritte zur oxidativen Kopplung von Methan zumindest teilweise zu Ethylen umgesetzt. Der oder die Schritte zur oxidativen Kopplung von Methan und der oder die nachgeschalteten thermischen Spaltschritte werden typischerweise in jeweils einem gemeinsamen Reaktor durchgeführt. Typischerweise erfolgt die Übertragung der Wärme in den oder die nachgeschalteten thermischen Spaltschritte dabei konvektiv, d.h. die Wärme wird mit dem strömenden Fluid aus einer Reaktionszone zur Durchführung des oder der Schritte zur oxidativen Kopplung von Methan in eine Reaktionszone zur Durchführung des oder der thermischen Spaltschritte überführt.

Ein wesentlicher Aspekt der vorliegenden Erfindung ist, dem oder den thermischen Spaltschritten, also dem oder den Post-Bed-Cracking-Schritten, Fluid des Trennabstroms, der aus dem Fluid des Dampfspaltabstroms gebildet wurde, und der überwiegend oder ausschließlich Ethan enthält, zuzuführen. Auf diese Weise lässt sich eine besonders vorteilhafte stoffliche Integration in einem entsprechenden kombinierten Verfahren erzielen, indem nämlich ein an sich wertloses Produkt eines Dampfspaltverfahrens an besonders günstiger Stelle weiter umgesetzt werden kann.

Während in herkömmlichen Anlagen zur Bearbeitung von Ethan als Recyclestrom oder Frischeinsatz separate Dampfspaltschritte durch Bereitstellung von Gasspaltöfen implementiert werden müssen, wie beispielsweise unter Bezugnahme auf die Figur 2A erläutert, können derartige Schritte in einem kombinierten Verfahren, wie es die vorliegende Erfindung vorschlägt, wegfallen. Das erfindungsgemäße Verfahren erlaubt also eine vorteilhafte stoffliche Nutzung des in dem oder den Dampfspaltschritten anfallenden Methans ohne großen zusätzlichen Aufwand. Weil, wie erwähnt, kein separater Gasspaltofen bereitgestellt werden muss, können die Verfahrensbedingungen für sämtliche Dampfspaltschritte (typischerweise sind in entsprechenden Verfahren mehrere parallel arbeitende Dampfspaltschritte vorgesehen) gleich gehalten werden. Dies verringert den Steuerungs- und/oder Regelaufwand beträchtlich.

Besonders vorteilhaft ist es, wenn im Rahmen der vorliegenden Erfindung unter Verwendung von Fluid des oder der Dampfspaltabströme ein weiterer Trennabstrom, der überwiegend oder ausschließlich Methan oder Methan und Wasserstoff enthält, gebildet wird. In diesem Fall kann Fluid des weiteren Trennabstroms als der oder einer der Reaktionseinsatzströme verwendet werden. Die Menge des entsprechend verwendeten Fluids richtet sich dabei insbesondere nach dem Methanbedarf des oder der Schritte zur oxidativen Kopplung von Methan. Durch einen Einsatz des Methans in entsprechenden Schritten kann eine weitere stoffliche Integration und eine aufwertende Nutzung des Methans erzielt werden. Diese ist im Allgemeinen vorteilhafter als eine Verfeuerung von Methan zur Beheizung. Gleichwohl kann auch im Rahmen der vorliegenden Erfindung anfallendes Methan zur Beheizung eingesetzt werden.

Eine weitere vorteilhafte Ausführungsform des erfindungsgemäßen Verfahrens umfasst, unter Verwendung von Fluid des oder der Dampfspaltabströme einen Pyrolyseöl- und/oder einen Pyrolysebenzinstrom zu bilden, wobei Fluid des Pyrolyseöl- und/oder des Pyrolysebenzinstroms ebenfalls zur Beheizung des oder der Dampfspaltschritte verwendet werden kann. Auf diese Weise lässt sich ein Minderangebot von Methan ausgleichen, wenn dieses dem oder den Schritten zur oxidativen Kopplung von Methan zugeführt wird. Eine entsprechende Beheizung kann direkt oder indirekt erfolgen. Unter einer direkten Beheizung wird dabei verstanden, dass das Fluid des Pyrolyseöl- und/oder des Pyrolysebenzinstroms direkt in einem Dampfspaltreaktor unterfeuert, d.h. verbrannt wird.. Es kann auch vorgesehen sein, durch eine Verbrennung des Fluids des Pyrolyseöl- und/oder des Pyrolysebenzinstroms Dampf zu erzeugen, mittels dieses Dampfs elektrische Energie zu erzeugen und den oder die Dampfspaltschritte elektrisch, also indirekt, zu beheizen.

Wenngleich zuvor betont wurde, dass im Rahmen der vorliegenden Erfindung vorteilhafterweise Ethan des Trennabstroms, der überwiegend oder ausschließlich Ethan enthält, in dem oder den thermischen Spaltschritten eingesetzt wird, kann selbstverständlich Fluid dieses Trennabstroms auch dem oder einem der Dampfspaltschritte unterworfen werden, insbesondere wenn ein Angebot an Ethan einen Bedarf des oder der thermischen Spaltschritte übersteigt. Auf diese Weise kann beispielsweise ein bereits vorhandener, für eine Speisung mit einem rückzuführenden Ethanstrom vorgesehener Ethan- bzw. Gasspaltofen beschickt und/oder das Fluid einem anderen Dampfspalteinsatzstrom zugespeist werden.

In letzterem Fall erweist es sich als besonders vorteilhaft, wenn eine Menge des Fluids des Trennabstroms, das dem oder den thermischen Spaltschritten zugeführt wird, und/oder eine Menge des Fluids des Trennabstroms, das dem oder einem der Dampfspaltschritte unterworfen wird, in Abhängigkeit von einem Ethanbedarf des oder der thermischen Spaltschritte und/oder des oder der Dampfspaltschritte eingestellt wird. Zu diesem Zweck können geeignete Steuer- und/oder Regeleinrichtungen vorgesehen sein, die eine besonders flexible und an den jeweiligen Bedarf angepasste Verfahrensführung ermöglichen.

Es ist besonders vorteilhaft, wenn Fluid des oder der Dampfspaltabströme und Fluid eines oder mehrerer Abströme des oder der thermischen Spaltschritte, die dem oder den Schritten zur oxidativen Kopplung von Methan nachgeschaltet sind, wenigstens einem gemeinsamen Aufbereitungs- und/oder Trennschritt unterworfen werden. Da in dem oder den Dampfspaltabströmen und dem oder den Abströmen des oder der thermischen Spaltschritte grundsätzlich chemisch vergleichbare Verbindungen enthalten sind, bietet eine gemeinsame Trennung besondere Vorteile und erlaubt einen Verzicht auf redundant bereitzustellende Trenneinrichtungen.

Besonders vorteilhaft ist es, wenn die Abwärme des oder der Schritte zur oxidativen Kopplung von Methan in einem gemeinsamen Reaktor eingesetzt wird, indem auch der oder die thermischen Spaltschritte durchgeführt werden. Entsprechende Reaktoren umfassen eine Katalysezone, die zur Durchführung der oxidativen Kopplung von Methan eingerichtet ist. Dieser Katalysezone ist in einer gemeinsamen baulichen Einheit eine Zone nachgeschaltet, in der die Abwärme der oxidativen Kopplung von Methan auf das gebildete Ethan einwirken kann. In diese nachgeschaltete Zone wird im Rahmen der vorliegenden Erfindung das Ethan des Trennabstroms eingespeist.

Eine Anlage zur Herstellung von Kohlenwasserstoffen ist ebenfalls Gegenstand der vorliegenden Erfindung. Eine derartige Anlage weist Mittel auf, die dafür eingerichtet sind, einen oder mehrere Dampfspalteinsatzströme, der oder die überwiegend ausschließlich Kohlenwasserstoffe mit zwei und mehr Kohlenstoffatomen enthalten, unter Gewinnung eines oder mehrerer Dampfspaltabströme einem oder mehreren Dampfspaltschritten zu unterwerfen. Ferner sind diese Mittel dafür eingerichtet, einen oder mehrere Reaktionseinsatzströme, die überwiegend oder ausschließlich Methan enthalten, unter Gewinnung eines oder mehrerer Reaktionsabströme, die Ethan enthalten, einem oder mehreren Schritten zur oxidativen Kopplung von Methan zu unterwerfen. Es sind ferner Mittel vorgesehen, die dafür eingerichtet sind, unter Verwendung von Fluid des oder der Dampfspaltabströme einen Trennabstrom, der überwiegend oder ausschließlich Ethan enthält zu bilden.

Erfindungsgemäß zeichnet sich eine derartige Anlage durch Mittel aus, die dafür eingerichtet sind, Fluid des oder der Reaktionseinsatzströme einem oder mehreren thermischen Spaltschritten zu unterwerfen, die dem oder den Schritten zur oxidativen Kopplung von Methan nachgeschaltet sind, und das Ethan, das in dem Fluid des oder der Reaktionsabströme enthalten ist, unter dem Einfluss von Abwärme des oder der Schritte zur oxidativen Kopplung von Methan zumindest teilweise zu Ethylen umzusetzen. Ferner sind dabei Mittel vorgesehen, die dafür eingerichtet sind, dem oder den thermischen Spaltschritten Fluid des Trennabstroms zuzuführen. Eine derartige Anlage profitiert von den Vorteilen, die zuvor erläutert wurden, und auf die daher ausdrücklich verwiesen wird.

Insbesondere weist eine derartige Anlage einen oder mehrere bereits erläuterte gemeinsame Reaktoren auf, die zur Durchführung des oder der Schritte zur oxidativen Kopplung von Methan und des oder der thermischen Spaltschritte eingerichtet sind.

Eine besonders vorteilhafte Anlage ist zur Durchführung eines Verfahrens eingerichtet, wie es zuvor ausführlich erläutert wurde.

Die Erfindung wird nachfolgend unter Bezugnahme auf die Zeichnungen näher erläutert, in denen eine bevorzugte Ausführungsform der Erfindung dargestellt ist.

Kurze Beschreibung der Zeichnung
Figur 1 zeigt ein Verfahren zur Erzeugung von Kohlenwasserstoffen gemäß einer Ausführungsform der Erfindung in Form eines schematischen Ablaufplans.
Figuren 2A und 2B veranschaulichen die Nachrüstung eines Verfahrens gemäß einer Ausführungsform der Erfindung in Form eines schematischen Ablaufplans.

### Ausführliche Beschreibung der Zeichnung

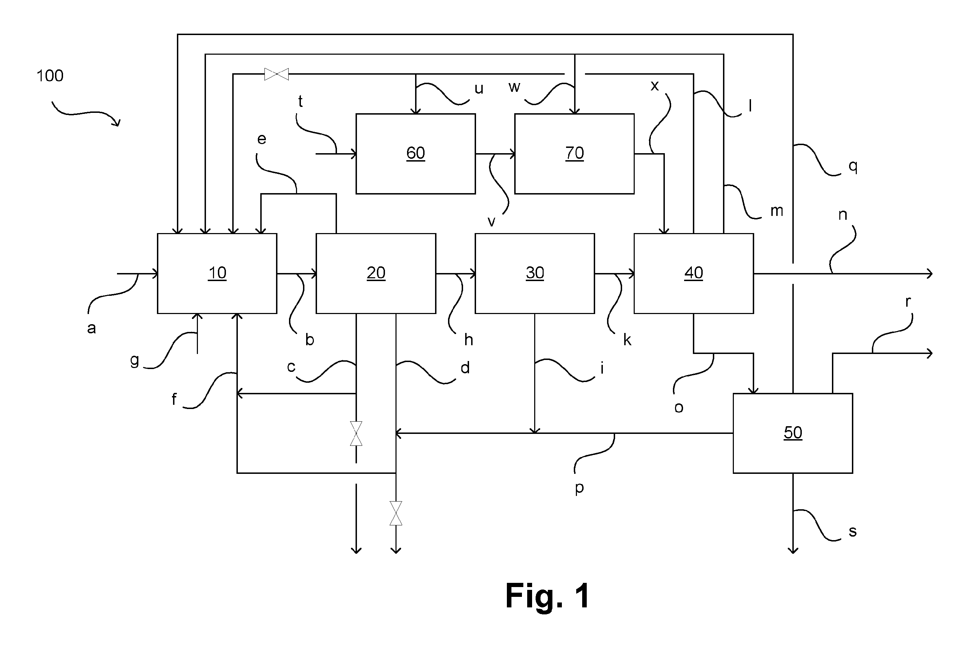

In Figur 1 ist ein Verfahren zur Erzeugung von Kohlenwasserstoffen gemäß einer Ausführungsform der Erfindung in Form eines schematischen Ablaufplans dargestellt und insgesamt mit 100 bezeichnet.

Ein oder mehrere Dampfspaltschritte, denen ein oder mehrere Dampfspalteinsatzströme a zugeführt werden, sind hier mit 10 bezeichnet. Ein oder mehrere in dem oder den Dampfspaltschritten 10 gebildete Dampfspaltabströme b werden zunächst einer Öl- und Wasserwäsche 20 zugeführt, wobei zunächst beispielsweise ein (Roh-)Pyrolyseölstrom c und ein (Roh-)Pyrolysebenzinstrom d abgetrennt werden. Ein Dampfstrom e kann in den oder die Dampfspaltschritte 10 zurückgeführt werden. Der Pyrolyseölstrom c und der Pyrolysebenzinstrom d können in beliebiger Weise aufbereitet und in Fraktionen getrennt werden. Es kann auch ein Strom f gewonnen werden, der zur Gewinnung von Wärmeenergie zumindest zeitweise in dem oder den Dampfspaltschritten 10 thermisch verwertet wird. Zusätzlich oder ausschließlich kann Wärmeenergie zumindest zeitweise über einen weiteren brennbaren Strom g bereitgestellt werden. Anteile der Ströme c und d können als Produkte aus dem Verfahren 200 ausgeleitet werden.

Ein nach der Öl- und Wasserwäsche 20 verbleibender Strom h kann einer Verdichtung und Trocknung 30 unterworfen werden, wo nochmals ein Pyrolysebenzinstrom i anfallen kann. Es verbleibt ein Strom k, der einer Tieftemperaturtrennung 40 zugeführt werden kann. In der Tieftemperaturtrennung 40 fallen mehrere Trennabströme, beispielsweise ein Brenngasstrom l, ein Ethanstrom m, ein Ethylenstrom n und ein Strom o aus Kohlenwasserstoffen mit drei und mehr Kohlenstoffatomen an. Der Strom o wird einer weiteren Trennung 50 unterworfen, in der ein weiterer Pyrolysebenzinstrom p, ein Propanstrom q, ein Propylenstrom r und ein Strom s aus Kohlenwasserstoffen mit vier Kohlenstoffatomen anfallen kann.

Der Brenngasstrom l, der Ethanstrom m und der Propanstrom q werden herkömmlicherweise in den oder die Dampfspaltschritte 10 zurückgeführt, wobei der Brenngasstrom l herkömmlicherweise verfeuert wird.

Das in Figur 1 veranschaulichte Verfahren 100 umfasst einen oder mehrere Schritte 60 zur oxidativen Kopplung von Methan, dem oder denen ein oder mehrere Reaktionseinsatzströme t zugeführt werden. Auch ein aus Fluid des Brenngasstroms m gebildeter Strom u kann als Reaktionseinsatzstrom den oder den Schritten 60 zur oxidativen Kopplung von Methan zugeführt werden. Der oder die Schritte 60 zur oxidativen Kopplung von Methan arbeiten in an sich bekannter Weise, so dass ein oder mehrere Reaktionsabströme v gebildet werden. Dem oder den Schritten 60 zur oxidativen Kopplung von Methan zugeführte weitere Ströme, beispielsweise ein oder mehrere Sauerstoffströme, sind nicht veranschaulicht.

Dem oder den Schritten 60 zur oxidativen Kopplung von Methan sind direkt ein oder mehrere thermische Spaltschritte 70 nachgeschaltet (sogenanntes Post-Bed Cracking). Wie erwähnt, fällt bei der oxidativen Kopplung von Methan eine große Abwärmemenge an, die sich vorteilhafterweise zur Spaltung beispielsweise des bei der oxidativen Kopplung von Methan enthaltenen Ethans einsetzen lässt. Die Abwärmemenge ist dabei so groß, dass eine größere Menge Ethan gespalten werden kann, als bei der oxidativen Kopplung von Methan gebildet wird. Wenngleich der oder die thermischen Spaltschritte 70 in der Figur 1 separat zu dem oder den Schritten 60 zur oxidativen Kopplung von Methan dargestellt sind, sei zu verstehen gegeben, dass diese typischerweise in einer einzigen baulichen Einheit implementiert sind.

Im Rahmen der in Figur 1 dargestellten Ausführungsform der Erfindung ist vorgesehen, Fluid des Ethanstroms m, also eines entsprechenden Trennabstroms aus der Tieftemperaturtrennung 40, dem oder den thermischen Spaltschritten 70 zuzuführen. Entsprechendes Fluid ist als Strom w veranschaulicht. Auf diese Weise lässt sich das Ethan gewinnbringend nutzen und es kann auf extern zugeführtes Ethan verzichtet werden. Gleichwohl kann bei Bedarf ein Teil des Stroms m auch in den oder die Dampfspaltschritte 10 zurückgeführt oder externes Ethan eingespeist werden.

Ein Abstrom x aus dem oder den thermischen Spaltschritten 70 kann beispielsweise in die Tieftemperaturtrennung 40 geführt werden. Auch eine Einspeisung in andere der Schritte, beispielsweise in die Verdichtung und Trocknung 30, ist möglich.

Die Figuren 2A und 2B veranschaulichen die Nachrüstung eines Verfahrens gemäß einer Ausführungsform der Erfindung in einem reinen Dampfspaltverfahren in Form eines schematischen Ablaufplans. Die dargestellten Ströme sind identisch zu den Strömen in Figur 1 bezeichnet. Die Figuren 2A und 2B sind nochmals stark vereinfacht.

In Figur 2A ist beispielsweise eine bestehende Anlage zur Herstellung von Kohlenwasserstoffen veranschaulicht, in der fünf parallele Dampfspaltschritte implementiert sind. Die mit 10 bezeichneten Dampfspaltschritte sind beispielsweise zur Umsetzung von Dampfspalteinsätzen a (nur an einer Stelle bezeichnet) vorgesehen, die einen großen Anteil Naphtha enthalten. Es werden jeweils Dampfspaltabströme b gewonnen (nur an einer Stelle bezeichnet). Ein mit 10' bezeichneter Dampfspaltschritt ist hingegen zur Umsetzung von Dampfspalteinsätzen vorgesehen, die überwiegend oder ausschließlich Ethan enthalten (sogenannter Gasspaltofen).

Die Dampfspaltabströme b werden einem hier stark schematisierten Aufbereitungs- und Trennschritt zugeführt, der beispielsweise die in Figur 1 dargestellten Schritte 10 bis 50 umfasst. Neben weiteren Trennabströmen werden beispielsweise ein Ethylenstrom n und ein Propylenstrom r gewonnen und als Produkte abgeführt. Ein ebenfalls als Trennabstrom gewonnener Ethanstrom m wird in den mit 10' bezeichneten Dampfspaltschritt geführt und dort umgesetzt. Zur Beheizung des mit 10' bezeichneten Dampfspaltschritts dient beispielsweise ein ebenfalls als Trennabstrom gewonnener Methanstrom l.

Gemäß Figur 2B ist der mit 10' bezeichnete Dampfspaltschritt durch einen oder mehrere Schritte 60 zur oxidativen Kopplung von Methan ersetzt, dem unter anderem der Methanstrom l ganz oder teilweise zugeführt wird. Einem oder mehreren nachgeschalteten thermischen Spaltschritten 70 wird der Ethanstrom ganz oder teilweise zugeführt, wie oben erläutert.

## Patentansprüche

1. Verfahren (100) zur Erzeugung von Kohlenwasserstoffen, bei dem ein oder mehrere Dampfspalteinsatzströme (a), die überwiegend oder ausschließlich Kohlenwasserstoffe mit zwei und mehr Kohlenstoffatomen enthalten, unter Gewinnung eines oder mehrerer Dampfspaltabströme (b) einem oder mehreren Dampfspaltschritten (10) unterworfen werden, und bei dem ein oder mehrere Reaktionseinsatzströme (t, u), die überwiegend oder ausschließlich Methan enthalten, unter Gewinnung eines oder mehrerer Reaktionsabströme (v), die Ethan enthalten, einem oder mehreren Schritten (60) zur oxidativen Kopplung von Methan unterworfen werden, wobei unter Verwendung von Fluid des oder der Dampfspaltabströme (b) ein Trennabstrom (m), der überwiegend oder ausschließlich Ethan enthält, gebildet wird, **dadurch gekennzeichnet, dass** Fluid des oder der Reaktionsabströme (v) einem oder mehreren thermischen Spaltschritten (70) unterworfen wird, die dem oder den Schritten (60) zur oxidativen Kopplung von Methan nachgeschaltet sind, und in denen das Ethan, das in dem Fluid des oder der Reaktionsabströme (v) enthalten ist, unter dem Einfluss von Abwärme des oder der Schritte (60) zur oxidativen Kopplung von Methan zumindest teilweise zu Ethylen umgesetzt wird, und dass dem oder den thermischen Spaltschritten (70) Fluid (w) des Trennabstroms (m) zugeführt wird.

2. Verfahren (100) nach Anspruch 1, bei dem unter Verwendung von Fluid des oder der Dampfspaltabströme (b) ein weiterer Trennabstrom (l), der überwiegend oder ausschließlich Methan oder Methan und Wasserstoff enthält, gebildet wird, wobei Fluid (u) des weiteren Trennabstroms (l) als der oder einer der Reaktionseinsatzströme verwendet wird.

3. Verfahren (100) nach einem der vorstehenden Ansprüche, bei dem unter Verwendung von Fluid des oder der Dampfspaltabströme (b) ein Pyrolyseöl- (c) und/oder ein Pyrolysebenzinstrom (d) gebildet wird, wobei Fluid des Pyrolyseöl- (c) und/oder des Pyrolysebenzinstroms (d) zur Beheizung des oder der Dampfspaltschritte (10) verwendet wird.

4. Verfahren (100) nach einem der vorstehenden Ansprüche, bei dem Fluid des Trennabstroms (m) dem oder einem der Dampfspaltschritte (10') unterworfen wird.

5. Verfahren (100) nach Anspruch 4, bei dem eine Menge des Fluids (w) des Trennabstroms (m), das dem oder den thermischen Spaltschritten (70) zugeführt wird, und/oder eine Menge des Fluids des Trennabstroms (m), das dem oder einem der Dampfspaltschritte (10') unterworfen wird, in Abhängigkeit von einem Ethanbedarf des oder der thermischen Spaltschritte (70) und/oder des oder der Dampfspaltschritte (10') eingestellt wird.

6. Verfahren (100) nach einem der vorstehenden Ansprüche, bei dem Fluid des oder der Dampfspaltabströme (b) und Fluid eines oder mehrerer Abströme (x) des oder der thermischen Spaltschritte (70), die dem oder den Schritten (60) zur oxidativen Kopplung von Methan nachgeschaltet sind, wenigstens einem gemeinsamen Aufbereitungs- und/oder Trennschritt (20 - 50) unterworfen wird.

7. Verfahren (100) nach einem der vorstehenden Ansprüche, bei dem das gesamte Fluid des oder der Reaktionsabströme (v) ungetrennt dem einen oder den mehreren thermischen Spaltschritten (70) unterworfen wird, die dem oder den Schritten (60) zur oxidativen Kopplung von Methan nachgeschaltet sind.

8. Verfahren (100) nach einem der vorstehenden Ansprüche, bei dem die Abwärme des oder der Schritte (60) zur oxidativen Kopplung von Methan in einem gemeinsamen Reaktor, in dem der oder die thermischen Spaltschritte (70) durchgeführt werden, eingesetzt wird.

9. Anlage zur Herstellung von Kohlenwasserstoffen, mit Mitteln, die dafür eingerichtet sind, einen oder mehrere Dampfspalteinsatzströme (a), die überwiegend oder ausschließlich Kohlenwasserstoffe mit zwei und mehr Kohlenstoffatomen enthalten, unter Gewinnung eines oder mehrerer Dampfspaltabströme (b) einem oder mehreren Dampfspaltschritten (10) zu unterwerfen, und einen oder mehrere Reaktionseinsatzströme (t, u), die überwiegend oder ausschließlich Methan enthalten, unter Gewinnung eines oder mehrerer Reaktionsabströme (v), die Ethan enthalten, einem oder mehreren Schritten (60) zur oxidativen Kopplung von Methan zu unterwerfen, wobei ferner Mittel vorgesehen sind, die dafür eingerichtet sind, unter Verwendung von Fluid des oder der Dampfspaltabströme (b) einen Trennabstrom (m), der überwiegend oder ausschließlich Ethan enthält, zu bilden, **gekennzeichnet durch** Mittel, die dafür eingerichtet sind, Fluid des oder der Reaktionsabströme (v) einem oder mehreren thermischen Spaltschritten (70) zu unterwerfen, die dem oder den Schritten (60) zur oxidativen Kopplung von Methan nachgeschaltet sind, und in denen das Ethan, das in dem Fluid des oder der Reaktionsabströme (v) enthalten ist, unter dem Einfluss von Abwärme des oder der Schritte (60) zur oxidativen Kopplung von Methan zumindest teilweise zu Ethylen umgesetzt wird, und Mittel, die dafür eingerichtet sind, dem oder den thermischen Spaltschritten (70) Fluid (w) des Trennabstroms (m) zuzuführen.

10. Anlage nach Anspruch 9, die zur Durchführung des oder der Schritte (60) zur oxidativen Kopplung von Methan und des oder der thermischen Spaltschritte (70) jeweils gemeinsame Reaktoren aufweist.

11. Anlage nach Anspruch 10, die zur Durchführung eines Verfahrens nach einem der Ansprüche 1 bis 8 eingerichtet ist.
